# EUROPÄISCHE PATENTSCHRIFT

(11) **EP 1 868 573 B1**
(45) Veröffentlichungstag und Bekanntmachung des Hinweises auf die Patenterteilung: **27.12.2017**
(21) Anmeldenummer: 06707491.4
(22) Anmeldetag: 09.03.2006
(51) Int. Cl.: A61K 9/14, A61K 9/10

(54) **VERFAHREN ZUR HERSTELLUNG ULTRAFEINER SUBMICRON-SUSPENSIONEN**
METHOD FOR PRODUCING ULTRAFINE SUBMICRONIC SUSPENSIONS
PROCEDE DE PRODUCTION DE SUSPENSIONS DE L'ORDRE DU MICRON ULTRAFINES

(30) Priorität: 11.03.2005 DE 102005011786
(43) Veröffentlichungstag der Anmeldung: 26.12.2007
(73) Patentinhaber: AbbVie Deutschland GmbH & Co KG, 65205 Wiesbaden (DE)
(72) Erfinder: MÖSCHWITZER, Jan, 1404 AK Bussum (NL)
(74) Vertreter: Uexküll & Stolberg
(86) Internationale Anmeldenummer: PCT/EP2006/002176
(87) Internationale Veröffentlichungsnummer: WO 2006/094808

(56) Entgegenhaltungen:
- WO-A-01/03670
- WO-A-96/32095
- WO-A-2004/060344
- DE-A1- 4 440 337
- US-A1- 2002 056 206

## Beschreibung

### 1. Gebiet der Erfindung

Die Erfindung beschreibt ein Verfahren zur Herstellung ultrafeiner Suspensionen im Submikronbereich für Bereiche der Pharmazie, Kosmetik, Lebensmittelherstellung, Agrar sowie technische Anwendungen.

### 2. Stand der Technik

Ungefähr 40% der pharmazeutischen Wirkstoffe, die sich in der Entwicklung oder in klinischen Phasen befinden, sind schwerlöslich, das heißt sie gehören zur Klasse der so genannten Problemarzneistoffe. Aufgrund ihrer schlechten Löslichkeit besitzen diese Arzneistoffe eine sehr geringe Bioverfügbarkeit nach oraler Gabe, häufig können die erforderlichen therapeutischen Blutkonzentrationsspiegel nicht erreicht werden. Eine Alternative wäre die intravenöse Gabe dieser Arzneistoffe, jedoch sind aufgrund der schlechten Löslichkeit der Wirkstoffe so große Injektionsvolumina erforderlich, dass diese in vielen Fällen nicht verabreicht werden können. Ein traditioneller Ansatz für diese Arzneistoffe ist die Erhöhung ihrer Sättigungslöslichkeit, z.B. durch Komplexierung (z.B. Cyclodextrin-Einschlussverbindungen (Uekama K. Design and Evaluation of Cyclodextrin-Based Drug Formulation. Chem Pharm Bull, Vol 52, 900-915 (2004) 2004;52(8):900-15)) oder - eine sehr einfache Möglichkeit - die Verwendung von injizierbaren Lösungsmittelmischungen (z.B. Wasser-Ethanol-Gemischen). Diese Ansätze lassen sich aber nur für wenige Arzneistoffe umsetzen, was man an der geringen Anzahl der auf dem Markt befindlichen Produkte sehen kann, die diese Technologien verwenden (z.B. Cyclodextrin-Verbindungen zur parenteralen Anwendung). Ein Problem dieser traditionellen Ansätze ist, dass das Molekül "passen" muß, das heißt, dass z.B. das schwerlösliche Arzneistoffmolekül in den Cyclodextrinring passen muß. Im Moment gewinnt diese Problematik immer mehr an Bedeutung, da sich die Zahl der schwerlöslichen Arzneistoffe ständig erhöht. Es gibt Schätzungen, dass ca. 60% der Arzneistoffe, die direkt aus der Synthese kommen, schwerlöslich sind (Merisko-Liversidge E. Nanocrystals: Resolving Pharmaceutical Formulation Issues associated with poorly water-soluble Compounds. In: Marty JJ, editor. Particles; 2002; Orlando: Marcel Dekker; 2002). Zusätzlich sind viele neue Wirkstoffe gleichzeitig schwerlöslich in Wasser und organischen Lösungsmitteln, das schließt von vornherein die Nutzung der traditionellen Formulierungsansätze, wie z.B. Lösungsmittelmischungen, aus. Darüber hinaus gibt es nur wenige organische Lösungsmittel, die zur parenteralen Verabreichung zugelassen sind, z.B. Ethanol und Propylenglykol. Die meisten Lösungsmittel besitzen eine zu hohe Toxizität.

Die oben beschriebenen Formulierungsansätze sind relativ spezifisch. Die ideale Lösung wäre ein universeller Formulierungsansatz, der für alle schwerlöslichen Wirkstoffe verwendet werden könnte. Die Mikronisierung ist ein solcher universeller Ansatz für peroral verabreichte Arzneistoffe, sie wird bereits seit vielen Jahren angewendet. Das Prinzip der Mikronisierung besteht in einer Vergrößerung der Oberfläche der Arzneistoffpartikel und in der damit einhergehenden Verbesserung der Auflösungsgeschwindigkeit. Aus diesem Grund ist die Mikronisierung ein universeller Ansatz für alle Arzneistoffe des biopharmazeutischen Klassifizierungssystems (engl.: biopharmaceutical specification class (BSC)) Klasse II (BSC II), also für Arzneistoffe, die nach peroaler Gabe zwar leicht permieren, deren Bioverfügbarkeit aber aufgrund einer langsamen Auflösungsgeschwindigkeit/geringen Sättigungslöslichkeit deutlich limitiert ist. Viele neu entwickelte Arzneistoffe sind derart schwerlöslich, das die durch Mikronisierung bewirkte Oberflächenvergrößerung nicht ausreicht, um die Auflösungsgeschwindigkeit zu erhöhen. Im Allgemeinen steht die Auflösungsgeschwindigkeit in Beziehung zur Sättigungslöslichkeit (Gesetz von Noyes-Whitney). Die Auflösungsgeschwindigkeit ist proportional zum Konzentrationsgradienten (cₛ-cₓ)/h (cₛ - Sättigungslöslichkeit, cₓ - Konzentration im umgebenden Medium, h - Dicke der Diffusionsschicht). Dass heißt, dass eine sehr geringe Sättigungslöslichkeit im Allgemeinen zu einer sehr geringen Auflösungsgeschwindigkeit führt.

Eine Konsequenz, um die Limitierungen der Mikronisierung zu überwinden, war der Schritt von der Mikronisierung zur Nanonisierung. Die Verringerung der Partikelgröße um eine weitere Dimension führt zu einer deutlichen Oberflächenvergrößerung und damit verbunden zu einer verbesserten Auflösungsgeschwindigkeit. Es sind verschiedenste Methoden beschrieben, um mikronisierte Wirkstoffkristalle in den Nanometerbereich zu überführen, d.h. um Nanokristalle herzustellen.

Naßmahlung mit Kugelmühlen wird angewendet, um die Größe von Arzneistoffkristallen dispergiert in Tensidlösungen zu reduzieren. Die Partikelgröße der "Makrosuspension" wird durch die Mahlkugeln reduziert. Ein Nachteil dieser Technologie ist die mögliche Kontamination des Produktes durch Abrieb von den Mahlkugeln (Buchmann S, Fischli, W., Thiel, F. P., Alex, R. Aqueous microsuspension, an alternative intravenous formulation for animal studies. In: 42 nd Annual Congress of the International Association for Pharmaceutical Technology (APV); 1996; Mainz; 1996. p. 124). Zusätzlich sind in Abhängigkeit von der Härte des Mahlgutes Mahlzeiten von Stunden bis Tagen erforderlich. Die erzielbaren Partikelgrößen sind in Abhängigkeit des Mahlgutes typischerweise unter 400 nm, häufig kann eine Partikelgröße von 200-300 nm erreicht werden. Um Partikelgrößen von ungefähr 100 nm zu erzielen, sind jedoch sehr lange Mahlzeiten erforderlich, was gleichzeitig mit einer stärkeren Kontamination des Produktes mit Mahlkugelabrieb einhergeht.

Eine alternative Herstellungsmethode ist die Verwendung von Hochdruckhomogenisatoren, also Methoden, die auf Kolben-Spalt-Prinzip oder dem Jet-Stream-Prinzip (Microfluidizer-Technologie, Microfluidics Inc. (US 6,018,080)) beruhen. Prinzip des Microfluidizers ist das frontale Aufeinanderprallen zweier Strahlen mit sehr großer Geschwindigkeit, wobei die Kollision der Partikel zu deren Zerkleinerung führt. Ein Nachtell dieser Methode ist, dass 50 oder mehr Zyklen erforderlich sind, um eine kleine Partikelgröße zu erzielen. Zusätzlich verbleibt im Fall von sehr harten, kristallinen Materialien ein relativ großer Anteil der Partikel mit einer Größe von mehr als einem *µ*m. Diese Partikel besitzen dann nicht die Vorteile von Nanokristallen, wie die signifikant gesteigerte Auflösungsgeschwindigkeit bei gleichzeitig erhöhter Sättigungslöslichkeit. Der Vorteil der gesteigerten Sättigungslöslichkeit tritt nur bei einer Partikelgröße kleiner als ca. 1-2 *µ*m in Erscheinung (Mosharraf, M., Nyström, C., The effect of particle size and shape on the surface specific dissolution rate of micronised practically insoluble drugs.Int. J. Pharm., 122, 35-47, 1995). Aus diesem Grund ist es von Vorteil, wenn mehr oder weniger alle Partikel eine Größe unterhalb dieses kritischen Wertes besitzen.

In einem Kolben-Spalt-Homogenisator wird die Makrosuspension durch einen sehr engen Spalt gepresst, der abhängig vom aufgewendeten Druck und von der Viskosität des Dispersionsmediums eine Größe von 5-20 *µ*m (Rainer H. Müller, Jan Möschwitzer and Faris Nadiem Bushrab, Manufacturing of nanoparticles by milling and homogenization techniques, eds. Gupta, Kompella, Publisher: Marcel Dekker, submitted for printing). Dabei führt die hohe Strömungsgeschwindigkeit zu Kavitationskräften, zusätzlich führen Partikelkollision sowie auftretende Scherkräfte ebenfalls zu einer Partikelzerkleinerung. Die Kolben-Spalt-Homogenisatoren werden genutzt, um Partikel dispergiert in reinen Wasser/Tensid-Mischungen zu homogenisieren (US 5,858,410), sie werden aber auch genutzt, um Partikel, die in nichtwässrigen Medien oder in Mischungen von Wasser mit wassermischbaren Flüssigkeiten dispergiert sind, zu homogenisieren (WO 0103670). Die mit Kolben-Spalt-Homogenisatoren erzieltbaren Partikelgrößen liegen dabei im Bereich von ca. 200-600 nm und im Fall von sehr harten Materialien im Bereich von ungefähr 700-900 nm (Muller RH, Jacobs C, Kayser O. Nanosuspensions as particulate drug formulations in therapy: Rationale for development and what we can expect for the future. Advanced Drug Delivery Reviews 2001;47(1):3-19;). Die Produktion von pharmazeutischen Wirkstoffen im Bereich von ca. 100 nm mit Hilfe der Kolben-Spalt-Homogenisatoren ist bis jetzt noch nicht beschrieben worden. Die minimal erzielbare Partikelgröße hängt von der Härte der Verbindungen, von der eingebrachten Leistungsdichte und von der Anzahl der Homogenisationszyklen ab. Bei einer bestimmten Leistungsdichte wird die minimale Partikelgröße nach einer bestimmten Zyklenzahl erreicht. Das wird durch die Tatsache erklärt, dass während der Zerkleinerung der Kristalle diese mehr und mehr perfekt werden, da ein Zerbrechen der Kristalle vorrangig an Fehlstellen auftritt und mit abnehmender Partikelgröße die Zahl der Fehlstellen demzufolge ständig reduziert wird. Das führt zu perfekteren Kristallen, die immer mehr aufzuwendende Kraft erfordern, um zerbrochen zu werden. Nach einer bestimmten Anzahl von Homogenisationsschritten mit einer bestimmten Leistungsdichte, reicht diese Leistungsdichte nicht mehr aus, um die relativ perfekten Kristalle weiter zu zerkleinern. Die Leistungsdichte wird bestimmt durch den aufgewendeten Homogenisationsdruck, typischerweise liegt dieser für die Produktion von Nanokristallen im Bereich von 1000-1500 bar. Es gab den Versuch, kleinere Nanokristalle durch die Aufwendung höherer Homogenisationdrücke von bis zu 4000 bar herzustellen, aber es wurde herausgefunden, dass eine weitere Erhöhung des Homogenisationsdruckes nur einen geringen Einfluss auf die erzielbare Partikelgröße hat. Es gab nur eine relative kleine Verringerung der Partikelgröße, als der Homogenisationsdruck auf 4000 bar erhöht wurde (Fichera, M.A., Wissing, S.A., Müller, R.H., Effect of 4000 bar homogenisation pressure on particle diminution on drug suspensions, Int. Meeting on Pharm., Biopharm. and Pharm. Technology, Nuremberg, 679-680, 2004). Bei diesem Beispiel führte eine Erhöhung des Homogenisationsdruckes von 1500 auf 4000 bar nur zu einer Verringerung der Partikelgröße um 165nm je 1000 bar Druckerhöhung. Im Gegensatz dazu konnte beim Erhöhen des Homogenisationsdruckes von 500 bar auf 1500 bar eine Reduktion der Partikelgröße um 547 nm festgestellt werden. Es scheint, dass es einen exponentiellen Zusammenhang zwischen der erforderlichen Erhöhung der Leistungsdichte zum Erzielen einer bestimmten Partikelgröße gibt. Zusätzlich nimmt die Abnutzung der eingesetzten Maschinen enorm zu, deshalb sind solch hohen Drücke für die industrielle Produktion von Nanokristallen ungeeignet. Zusammenfassend kann man feststellen, dass - wenn man die Bedürfnisse in der pharmazeutischen Produktion oder in anderen Bereichen berücksichtigt - die Homogenisationsdrücke im Bereich von 1000-2000 bar liegen sollten. Aus diesem Grund mussten neue Ansätze gefunden werden, um kleinere Partikelgrößen zu erzielen. Es gibt eine vorherrschende Literaturmeinung, dass eine Erhöhung des Homogenisationsdruckes keine Lösung darstellt, um zu kleineren Nanokristallen zu gelangen, zusätzlich beschreiben alle bisher veröffentlichten Daten immer nur eine durch Homogenisation erzieltbare Partikelgröße von größer 200nm.

Ein sehr alter Ansatz zur Herstellung von Nanokristallen ist die Präzipitation, in den alten Arzneibüchern als "via humida paratum" (auf flüssigem Wege bereitet) beschrieben. Dabei wird der Wirkstoff in einem Lösungsmittel gelöst, diese Lösung wird schnell zu einem Nicht-Lösungsmittel (welches mit dem Lösungsmittel mischbar ist) gegeben, wobei der Wirkstoff ausfällt.

Die Partikel werden im Allgemeinen mit Tensiden oder polymeren Stabilisatoren stabilisiert. Dieses Prinzip wurde angewendet, um die sogenannten "Hydrosole" herzustellen (US 5,389,382). Später wurden einige Modifikationen dieses Präzipitationsprinzipes beschrieben (US 6,251,945). Es gibt aber einige Probleme, die mit dem Präzipitationsprinzip verbunden sind. Das Hauptproblem ist, die präzipitierten Kristalle in Nanometerbereich zu stabilisieren. Die Nanokristalle versuchen zu wachsen und Mikrokristalle zu bilden. Ein Ansatz, dies zu verhindern, ist die sofortige Trocknung der hergestellten Suspension, z.B. durch Lyophilisation (Sucker, H., Hydrosole - eine Alternative für die parenterale Anwendung von schwer wasserlöslichen Wirkstoffen, in: Müller, R. H., Hildebrand, G. E., (Hrsg.), Pharmazeutische Technologie: Moderne Arznei-formen, 2. Auflage, 1998, WVG, Stuttgart)). Ein alternativer Ansatz ist die Fällung der Partikel mit anschließendem Eintrag von Energie (z.B. durch Scherkräfte oder Ultraschall (US 6,607,784). Diese Kräfte können z.B. durch Hochgeschwindigkeitsmischer oder verschiedene Hochdruckhomogenisatoren (z.B. Geräte der Firmen APV Gaulin, NiroSoavi, Avestin) oder im Fall der Ultraschallverwendung durch Geräte der Fima Sonics aufgebracht werden. Die Behandlung mit Kräften führt dabei zu einer Stabilisierung der hergestellten Partikel, die Kristalle verändern ihre Größe während der Lagerung nicht, im Gegensatz zu den Kristallen, die nicht mit Scherkräften behandelt wurden. Dadurch gelten für diese Methode bezüglich der minimal erreichbaren Partikelgröße die gleichen Beschränkungen wie für die oben beschriebenen Methoden der Hochdruckhomogenisation (US 5,858,410/ WO0103670). Zusätzlich ist es bei der Anwendung dieser Präzipitationsmethode - zumindest in den meisten Fällen - erforderlich, dass Lösungsmittel zu entfernen. Im Allgemeinen ist aber jedes Lösungsmittel zumindest in einem bestimmten Umfang in dem Nicht-Lösungsmittel (z.B. Wasser) löslich, das bedeutet, dass immer ein gewisser Restgehalt im Wasser zurückbleibt. Außerdem kann das Entfernen des Lösungsmittels zu einer Aggregation der Nanokristalle oder sogar zum Partikelwachstum führen. Deshalb wäre ein Zerkleinerungs- oder Herstellungsprozess wünschenswert, der die Verwendung von Lösungsmitteln ausschließt.

Ferner ist aus der WO 2004/060344 A2) ein Verfahren zur Herstellung einer pharmazeutischen Zusammensetzung in Form einer trockenen Pulvermischung bekannt, bei dem einen pharmazeutischen Wirkstoff enthaltende Mikropartikel mit einer Größe von 1 bis 50 *µ*m durch Sprühtrocknung und anschließende Deagglomerierung mittels Strahlmahlung gebildet werden.

Die WO 2002/056206 A1 offenbart ein Verfahren zur Herstellung von pharmazeutischem Inhalationspulver, das kristalline Partikel einer Inhalationsverbindung umfasst. Bei dem Verfahren wird eine Inhalationsverbindung in einem Lösungsmittel gelöst und diese Lösung in Tropfenform oder als Düsenstrahl in ein Nicht-Lösungsmittel eingeführt, das mit dem Lösungsmittel mischbar ist. Trocknung kann durch Sprühtrocknung erfolgen.

Ferner offenbart die US 2002/0056206 A1 ein Sprühtrocknungsverfahren für die Herstellung kleiner Partikel oder Mikropartikel, die den Wirkstoff Fenofibrat und eine Phospholipidverbindung enthalten. Bei dem Verfahren werden der Wirkstoff und das Phospholipid unter hoher Scherung in einem wässrigen Trägermaterial innerhalb eines ersten Temperaturbereichs am oder oberhalb des Schmelzpunkts des Wirkstoffs gemischt, die heiße Suspension homogenisiert und das heiße Homogenisat sprühgetrocknet.

Außerdem offenbart die bereits erwähnte WO 01/03670 A1 die Hochdruckhomogenisation einer Suspension zur Erzielung eine Größenreduktion der suspendierten Partikel in den Mikro- und Nanobereich. Das feste Ausgangsmaterial ist zu einem Pulver zermahlen worden und anschließend zur weiteren Verarbeitung dispergiert worden.

Auch die DE 44 40 337 A1 beschreibt die Herstellung einer Nanopartikelsuspension mittels Anwendung von Hockdruckhomogenisation auf ein festes pulverförmiges Ausgangsmaterial.

Arzneistoffnanokristalle besitzen zwei besonders vorteilhafte physikalische Eigenschaften, zum einen die gesteigerte Sättigungslöslichkeit und zum anderen die vergrößerte Oberfläche.

Beide Eigenschaften führen zu einem Anstieg der Auflösungsgeschwindigkeit nach dem Gesetz von Noyes-Whitney. Im Allgemeinen ist es vorteilhaft, Nanopartikel so klein wie möglich zu verwenden, um eine maximale Verbesserung der oralen Bioverfügbarkeit oder eine sehr schnelle Auflösungsgeschwindigkeit im Blut nach intravenöser Verabreichung zu erzielen. Um solch kleine Nanopartikel mit den bis dato bekannten Methoden herzustellen, ist eine relativ große Anzahl von Homogenisationszyklen erforderlich. Das ist nicht vorteilhaft für den Produktionsprozess. Eine möglichst geringe Zyklenzahl ist ideal, um die Produktionskosten zu senken und eine Kontamination des Produktes mit Geräteabrieb zu minimieren. Aus diesem Grund wäre es vorteilhaft, die Anzahl der benötigten Zyklen deutlich zu senken. Außerdem kann im Fall von sehr harten Arzneistoffen eine Reduktion der Partikelgrößen in den Nanometerbereich bisher kaum oder gar nicht erreicht werden. Alle aufgeführten Aspekte wurden in der vorliegenden Erfindung realisiert.

### 3. Beschreibung der Erfindung

Die vorliegende Erfindung betrifft ein Verfahren, das einen sehr schnellen Trocknungsschritt eines Produktes mit der anschließenden Einwirkung von mittleren oder hohen Scherkräften auf dieses Produkt verbindet. Der Wirkstoff wird in einem geeigneten Lösungsmittel, in dem er eine gute Löslichkeit besitzt, gelöst. Das Lösungsmittel kann - aber muss nicht - zusätzliche Stoffe, wie z.B. Tenside, Polymere, polymerische Stabilisatoren, Kohlenhydrate, Elektrolyte oder Nichtelektrolyte enthalten.

Das erfindungsgemäße Verfahren zur Herstellung ultrafeiner Suspensionen ist dadurch gekennzeichnet, dass
- eine bei 20°C als Feststoff vorliegende Substanz in einem Lösungsmittel gelöst wird,
- dem sich im Trocknungsvorgang befindlichen Anteil dieser Lösung sehr schnell innerhalb von 5 Sekunden das Lösungsmittel im Wesentlichen entzogen wird,
- das dabei erhaltene Pulver, dessen Partikel eine durchschnittliche Partikelgröße im Bereich von 1 bis 50 *µ*m (gemäß Laserdiffraktometrie) aufweisen, in einem Dispersionsmedium dispergiert wird und
die so erhaltene Suspension unter Anwendung von hohen Kräften 10⁹ bis 10¹³ W/m³ zu einer Suspension weiterverarbeitet wird, deren Feststoffpartikel eine durchschnittliche Partikelgröße (gemäß Photonenkorrelationsspektroskopie PCS) unter 1 *µ*m (1000 nm), und insbesondere unter 400 nm, speziell unterhalb von 200 nm aufweisen.

Beispiele für Lösungsmittel sind: N-Methyl-2-pyrrolidinon, 2-Pyrrolidon, Dimethylacetamid, Milchsäure, Ethanol, Methanol, Isopropanol, Aceton, Chloroform, Dichlormethan, Dimethylsulfoxid, N-Propanol, Glycerol, Ethylenglycol, Dimethylformamid, Dimethylacetamid, organische Säuren (z.B. Essigsäure, Ameisensäure, Fumarsäure).

In einigen Fällen ist es wünschenswert, Wirkstoffe in nanokristalliner Form vorliegen zu haben, die in nichtwässrigen Medien schwerlöslich sind. Diese Wirkstoffe können in Wasser löslich sein, sollen aber für ihre Anwendung in einem nichtwässrigen Medium in nanokristalliner Form dispergiert vorliegen. Diese Wirkstoffe würden in Wasser gelöst werden, die Lösung würde einem sehr schnellen Trocknungsschritt unterzogen werden und anschließend würde dieses Produkt in einem nichtwässrigen Medium weiterverarbeitet werden, in welchem der Wirkstoff eine geringe Löslichkeit besitzt.

Typische Tenside oder stabilisierende Substanzen, die dem Lösemittel zugesetzt werden können, sind z.B. Verbindungen aus der Reihe der Poloxamere, Poloxamine, ethoxylierten Mono- und Diglyceride, ethoxylierten Lipide und Lipoide, ethoxylierten Fettalkohole und Alkylphenole, ethoxylierten Fettsäureester, Polyglycerinether und -ester, Lecithine, Ester und Ether von Zuckern oder Zuckeralkoholen mit Fettsäuren oder Fettalkoholen, Phospholipide und Sphingolipide, Sterine, deren Ester oder Ether sowie deren Mischungen dieser Verbindungen. Daneben kommen auch Eilecithin, Sojalecithin oder hydrierte Lecithine, deren Mischungen oder Mischungen aus einem oder beiden Lecithinen mit einer oder mehreren Phopholipidkomponenten, Cholesterin, Cholesterinpalmitat, Stigmasterin oder andere Sterine in Frage, um der Lösung zugesetzt zu werden.

Unter Umständen kann es erforderlich sein, der Lösung weitere Substanzen zuzusetzen, um die Eigenschaften der Lösung selbst oder die Eigenschaften des aus der Lösung hergestellten trockenen Pulvers zu beeinflussen. Dazu kommen unter anderem in Frage: Diacetylphosphat, Phosphatidylglycerol, gesättigte oder ungesättigte Fettsäuren, Natriumcholat, Peptisatoren oder Aminosäuren, sowie Celluloseether und -ester, Polyvinylderivate, Alginate, Xanthane, Pektine, Polyacrylate, Poloxamere und Poloxamine, Polyvinlyalkohol, Polyvinylpyrrolidon oder Glucose, Mannose, Trehalose, Mannit und Sorbit, Fructose, Natriumcitrat, Natriumhydrogenphosphat, Natriumdihydrogenphosphat, Natriumchlorid, Kaliumchlorid, Glycerin. Wenn erforderlich können dem Lösungsmittel auch Farbstoffe, entweder in gelöster Form oder in unlöslicher Form als Pigmente, zugesetzt werden.

Die Konzentrationen solcher Komponenten liegen pro Komponente, bezogen auf das Gesamtgewicht der Lösung, bevorzugt im Bereich von 1 bis 90%, insbesondere von 1 bis 20% und bevorzugt unterhalb von 10% liegen, idealerweise unterhalb von 0,01 bis 5%.

Diese Lösung, die einen oder mehrere Wirkstoffe und einen oder mehrere Hilfsstoffe enthalten kann, wird dann weiterverarbeitet. Dazu wird sie einem sehr schnellen Trocknungsprozess, beispielsweise mit Hilfe eines kommerziell verfügbaren Sprühtrockners, unterzogen. Beispiele für solche Sprühtrockner sind Geräte der Firmen Niro, Nubilosa, Caldyn, Büchi, APV, Trema etc.

Sprühtrocknung ist als ein Prozess bekannt, der sowohl für die Verarbeitung von thermolabilen als auch von thermostabilen Wirkstoffen angewendet werden kann. Besonders durch die Verwendung von Lösungsmitteln mit niedrigem Siedepunkt können auch sehr temperaturempfindliche Stoffe verarbeitet werden.

Im Gegensatz zu den in der Literatur beschriebenen Sprühtrocknungsmethoden zu Herstellung kleiner und kleinster Partikel (US 6,565,885; US 6,696,084, PCT/US00/34606), wird das hier gewonnene Material durch die Anwendung von Scherkräften weiterverarbeitet. Diese Scherkräfte führen zu einem Produkt im Nanometerbereich, welches gleichzeitig eine relativ geringe Partikelgrößenverteilung aufweist.

Im Fall von oxidationsempfindlichen Stoffen kann sowohl der Trocknungsprozess als auch die anschließende Anwendung der Scherkräfte unter Schutzgasatmosphäre erfolgen.

Alternativ können z.B. auch Wirbelschichttrockner (z.B. Geräte der Firma Niro) oder sonstige Geräte (wie z.B. Dünnschichtvakuumtrockner oder Trockenwalzentrockner) verwendet werden, die zu einer schnellen Trocknung durch Entziehen des Lösemittels führen. Es ist lediglich erforderlich, dass der Lösemittelentzug relativ schnell auftritt.

Allgemein ist festzustellen, daß die erfindungsgemäß angewendeten hohen Kräfte Scher-, Kavitations-, Mahl- und/oder Ultraschallkräfte sind, insbesondere Hochdruckhomogenisatoren, Jet-Stream-Geräte, Kugelmühlen, Hochscherungsmischer oder Ultraschallapparaturen sind, wobei das eingesetzte Gerät mit einer Leistungsdichte von 10⁹ bis 10¹³ W/m³ arbeitet, wobei die hohen Kräfte vorzugsweise im Bereich von 10⁹ oder 10¹⁰ bis 10¹² oder 10¹³ W/m³ liegen.

Das erfindungsgemäße Verfahren zeichnet sich dadurch aus, daß der Lösungsmittelentzug/die Trocknung des in dem Trocknungsvorgang befindlichen Anteil der hergestellten Lösung innerhalb von 5 Sekunden, vorzugsweise innerhalb von 2, insbesondere von 1, bevorzugter 0,5, und noch bevorzugter innerhalb von 0,1 Sekunden erfolgt.

Ferner zeichnet sich das erfindungsgemäße Verfahren dadurch aus, daß der Lösungsmittelentzug/die Trocknung zu einem Restlösungsmittelgehalt/Restfeuchtigkeit von maximal 10 Gew.%, bevorzugt 5 Gew.%, bevorzugter 1 Gew.%, insbesondere 0,5 Gew.% spezieller 0,1 Gew.% oder darunter führt.

Die zu verarbeitenden Wirkstoffe können aus verschiedensten Bereichen stammen, d.h. es können pharmazeutische Wirkstoffe, kosmetische Wirkstoffe, aber auch Zusatzstoffe für die Nahrungsmittelindustrie sowie Materialen für andere technische Bereiche verarbeitet werden, die bevorzugt als nanokristallines Material vorliegen sollen, wie z.B. Farbstoffe und Farbstoffpigmente für Farben und Lacke oder für kosmetische Anwendungen.

Pharmazeutische Wirkstoffe können aus den nachfolgend aufgeführten therapeutischen Gebieten stammen (ggf. in Form ihrer wenig wasserlöslichen Form, z. B. als Base anstelle des Hydrochlorids).

Beispiele für in Form einer Nanosuspension zu verarbeitende Arzneistoffgruppen sind:
1. Analgetika/Antirheumatika
   z.B. Morphin, Codein, Piritramid, Fentanyl, Levomethadon, Tramadol, Diclofenac, Ibuprofen, Dexibuprofen, Ketoprofen, Dexketoprofen, Meloxicam, Indometacin, Naproxen, Piroxicam, Rofecoxib, Celecoxib,
2. Antiallergika
   z.B. Pheniramin, Dimetinden, Terfenadin, Astemizol, Loratidin,Desloratadin, Doxylamin, Meclozin, Fexofenadin, Mizolastin
3. Antibiotika/Chemotherapeutika
   z.B. Rifamoicin, Ethambutol, Thiazetazon, Buparvaquon, Atovaqon, Tarazepid
4. Antiepileptika
   z.B. Carbamazepin, Clonazepam, Mesuximid, Phenytoin, Valproinsäure
5. Antimykotika
   a) intern:
      z.B. Natamycin, Amphotericin B, Miconazol, Itraconazol
   b) extern ausserdem:
      z.B. Clotrimazol, Econazol, Fenticonazol, Bifonazol, Ketoconazol, Tolnaftat
6. Corticoide (Interna)
   z.B. Aldosteron, Fludrocortison, Betametason, Dexametason, Triamcinolon, Triamcinolonacetonid, Fluocortolon, Hydrocortison, Hydrocortisonacetat, Prednisolon, Prednyliden, Cloprednol, Budesonid, Methylprednisolon
7. Dermatika
   a) Antibiotika:
      z.B. Tetracyclin, Erythromycin, Framycetin, Tyrothricin, Fusidinsäure
   b) Virustatika wie oben, ausserdem:
      z.B. Vidarabin
   c) Corticoide wie oben, ausserdem:
      z.B. Amcinonid, Flupredniden, Alclometason, Clobetasol, Halcinonid, Fluocinolon, Clocortolon, Flumetason, Diflucortolon, Fludroxycortid, Halometason, Desoximetason, Fluocinolid, Fluocortinbutyl, Flupredniden, Prednicarbat, Desonid
8. Hypnotika, Sedativa
   z.B. Cyclobarbital, Pentobarbital, Methaqualon, Benzodiazepine (Flurazepam, Midazolam, Nitrazepam, Lormetazepam, Flunitrazepam, Triazolam, Brotizolam, Temazepam, Loprazolam)
9. Immuntherapeutika und Zytokine
   z.B. Azathioprin, Ciclosporin
13. Lokalanaesthetika
   a) intern:
      z.B. Butanilicain, Mepivacain, Bupivacain, Etidocain, Lidocain, Articain
   b) extern ausserdem:
      z.B. Oxybuprocain, Tetracain, Benzocain
10. Migränemittel
   z.B. Lisurid, Methysergid, Dihydroergotamin, Ergotamin, Triptane (wie z.B. Zolmitriptan, Sumatriptan, Rizatriptan)
11. Narkosemittel
   z.B. Methohexital, Propofol, Etomidat, Ketamin, Thiopental, Droperidol, Fentanyl
12. Nebenschilddrüsenhormone, Calciumstoffwechselregulatoren z.B. Dihydrotachysterol
13. Ophthalmika
   z.B. Cyclodrin, Cyclopentolat, Homatropin, Tropicamid, Pholedrin, Edoxudin, Aciclovir, Acetazolamid, Diclofenamid, Carteolol, Timolol, Metipranolol, Betaxolol, Pindolol, Bupranolol, Levobununol, Carbachol
14. Psychopharmaka
   z.B. Benzodiazepine (Lorazepam, Diazepam), Clomethiazol
15. Sexualhormone und ihre Hemmstoffe
   z.B. Anabolika, Androgene, Antiandrogene, Gestagene, Estrogene, Antiestrogene
16. Zytostatika und Metastasehemmer
   a) Alkylantien wie Melphalan, Carmustin, Lomustin, Cyclophosphamid, Ifosfamid, Trofosfamid, Chlorambucil, Busulfan, Prednimustin, Thiotepa
   b) Antimetabolite wie Fluorouracil, Methotrexat, Mercaptopurin, Tioguanin
   c) Alkaloide wie Vinblastin, Vincristin, Vindesin
   d) Antibiotoka wie Dactinomycin
   e) Taxol und verwandte bzw. analoge Verbindungen
   f) Dacarbazin, Estramustin, Etoposid

Pharmazeutische Wirkstoffe von besonderem Interesse sind Oxaiplatin, Paclitaxel, Taxane, Ketoconazol, Itraconazol, Ibuprofen, Naproxen, Omeprazol, Pantoprazol, Loratadin, Desloratadin, Loperamid, Daglutril.

Die so erhaltenen trockenen Pulver, die nach der oben beschriebenen Methode hergestellt wurden, besitzen eine Partikelgröße im Bereich von ca. 1*µ*m bis 10*µ*m, manchmal bis etwa 30-50*µ*m. In den meisten Fällen ist das erhaltene Produkt kristallin oder fast kristallin (Beispiele 8-11). Abhängig von den chemischen Eigenschaften und dem Schmelzpunkt der Wirkstoffe, kann das trockene Produkt auch teilweise amorph oder amorph sein.

Anschließend wird das erhaltene Pulver in einem Nichtlösemittel mittels herkömmlicher Rührmethoden dispergiert, z.B. durch eine Hochgeschwindigkeitsrührscheibe. Die dabei aufzuwendenden Rührkräfte sind relativ gering und sollen gerade ausreichen, um das trockene Produkt in eine Suspension zu überführen. Wenn notwendig, können die Partikel durch die Zugabe von Tensiden, Antiflokkulantien (z.B. Natriumcitrat) und polymere Stabilisatoren, wie bereits oben beschrieben, stabilisiert werden. Es können also der aus dem Pulver herzustellenden Lösung einzelne oder mehrere Stoffe der oben genannten Beispiele zugesetzt werden, um die Partikeldispersionen in gewünschter Form zu beeinflussen.

Auf die so hergestellten Suspensionen werden dann hohe Schwer- und/oder Kavitationskräfte angewendet. Es werden Geräte mit einer Leistungsdichte im Bereich von 10⁹ / 10¹³ W/m³ verwendet, mit deren Hilfe dann hohe Kräfte auf die Suspensionen aufgewendet werden können. Beispiele für solche Geräte sind Strahl-Homogenisatoren oder Kolben-Spalt-Homogenisatoren (z.B. Geräte der Serien Avestin, APV Gaulin, Niro Soavi) oder Ultraschallerzeuger der Firma Sonics.

Methoden, die in der Literatur beschrieben werden, wie z.B. die Hydrosol-Methode (Sucker, H., Hydrosole - eine Alternative für die parenterale Anwendung von schwer wasserlöslichen Wirkstoffen, in: Müller, R. H., Hildebrand, G. E., (Hrsg.), Pharmazeutische Technologie : Moderne Arzneiformen, 2. Auflage, 1998, WVG, Stuttgart) und die Micropräzipitationsmethode (US 6,607,784) erfordern, dass das Lösungsmittel, welches zum Auflösen des Wirkstoffes verwendet wird, mit dem Dispersionsmedium mischbar ist, welches für die Herstellung der Nanosuspension Anwendung findet. Diese Voraussetzungen brauchen bei der hier beschriebenen Erfindung nicht erfüllt sein, es kann nahezu jedes Lösungsmittel verwendet werden, in welchem der zu verarbeitende Wirkstoff gelöst werden kann. Das verwendete Lösungsmittel muss nicht mit dem Dispersionsmedium zur Herstellung der Nanosuspension mischbar sein. Das eröffnet außerdem auch die Möglichkeit der Verwendung relativ toxischer Lösungsmittel, wie z.B. Chloroform oder Dimethylsulfoxid, da diese durch oder nach dem schnellen Trocknungsprozess sehr effizient entfernt werden können. Die zurückbleibenden Restkonzentrationen bewegen sich im unteren ppm-Bereich und werden bei der Herstellung der Nanosuspension noch weiter verdünnt. Das führt zu einer insgesamt viel geringeren Kontamination mit Lösungsmitteln, als dies bei der Anwendung der bisher bekannten Methoden möglich ist.

Durch die Anwendung der Hochdruckhomogenisation auf eine wässrige Suspension des Arzneistoffs Ibuprofen konnte kein nanokristallines Material erhalten werden. Die Partikelgröße, die mit Hilfe der Photonenkorrelationsspektroskopie bestimmt wurde, lag bei 1172 nm (Beispiel 1). Sogar eine Erhöhung der Zyklenzahl auf 60 Homogenisationszyklen führte nur zu einer vernachlässigbar kleineren Zerkleinerung auf 1150nm. Das heißt, dass man im Fall von relativ harten Materialien bei Anwendung der bisher bekannten Methoden nicht in der Lage ist, leicht Arzneistoffnanokristalle herzustellen. Dann wurde Ibuprofen nach der erfindungsgemäßen Methode hergestellt. Dazu wurde es sprühgetrocknet (Beispiel 8) und das sprühgetrocknete Pulver in einer Tensidlösung dispergiert (Beispiel 2). Bereits nach 20 Homogenisationszyklen konnten Arzneistoffnanokristalle mit einer Größe von 636 nm erzielt werden. Der Polydispersitätsindex (PI) von 0.169 zeigte eine relative enge Partikelgrößenverteilung an, die im Bereich von Emulsionen für parenterale Ernährung lag (Müller R.H., Heinemann, S. Surface Modelling of Microparticles as Parenteral Systems with High Tissue Affinity. In: Gurny, R., Juninger, H. E., ed. Bioadhesion - Possibilities and Future Trends: Wissenschaftliche Verlagsgesellschaft Stuttgart; 1989, 202-14). Die Anwendung der Erfindung führte also bei identischer oder ähnlicher Zyklenzahl zu einer Halbierung der erreichten Partikelgröße im Vergleich zur bekannten Methode.

Für bestimmte Anwendungen werden größere Nanokristalle bevorzugt, z.B. um verlängerte Blutspiegel nach peroraler Applikation zu erzielen. Zu feine Nanokristalle würden sich zu schnell auflösen, aus diesem Grund werden manchmal Nanokristalle im oberen Nanometerbereich bevorzugt. Mit dem hier dargestellten Prozess war man in der Lage, Nanokristalle mit einer Größe von 930 nm innerhalb von 5 Homogenisationszyklen herzustellen (Beispiel 2). Das ist für eine industrielle Produktion sehr vorteilhalft.

In der Literatur wird postuliert, dass die Partikelgrößenverkleinerung bevorzugt bei der Verwendung von amorphen Stoffen als Ausgangsmaterial auftritt. Das hier verwendete sprühgetrocknete Ibuprofen war Ergebnissen der Diffential Scanning Calorimetry (DSC) aber eindeutig kristallin (Beispiel 3).

Aus diesem Grund kann die überraschende Effizienz der Partikelgrößenverkleinerung nicht durch die Verwendung von amorphem Ausgangsmaterial begründet werden.

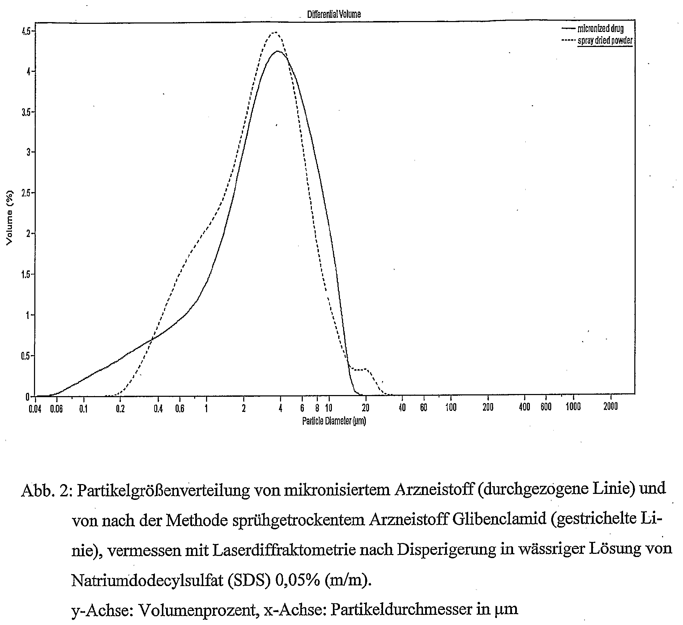

In der Literatur wird weiterhin beschrieben, dass die Verwendung von mikronisiertem Ausgangsmaterial die Partikelgrößenverkleinerung begünstigt, dass heißt zu kleineren Partikeln nach weniger Homogenisationszyklen führt. Das für die Sprühtrocknung verwendete Glibenclamid wurde bezüglich der Partikelgröße analysiert (Beispiel 6), dann wurde dieses Pulver in Ethanol (96% v/v) gelöst und sprühgetrocknet. Dieses sprühgetrocknete Pulver wurde wiederum bezüglich der Partikelgröße untersucht. Für die Partikelgrößenbestimmung wurde die Laserdiffraktometrie angewendet. Dazu wurden die trockenen Pulver, sowohl das Ausgangsmaterial als auch das sprühgetrocknete Produkt, in tensidhaltigem Wasser dispergiert und mit Hilfe eines Coulter LS 230 (Coulter Electronics, USA) analysiert. Abbildung 2 in Beispiel 6 zeigt, dass die Partikelgrößenverteilungen beider Pulver nahezu identisch sind. Aus diesem Grund kann die überraschende Partikelgrößenreduktion, die bei dem neuen Prozess auftritt, nicht mit einer unterschiedlichen Größe des Ausgangsmaterials begründet werden. Zusammenfassend kann man feststellen, dass weder ein amorpher Charakter des Ausgangsmaterials noch eine geringere Größe des Ausgangsmaterials eine Rolle spielten. Diese Faktoren begründen nicht die erzielten Zerkleinerungseffekte.

Glibenclamid wurde zunächst nach der bereits bekannten Methode hergestellt (US 5,858,410) (Beispiel 4, Charge Glb-A). Dafür wurde das pulverisierte Ausgangsmaterial in einer Tensidlösung (enthielt 1% Poloxamer 188 und 0.2% Natriumdodecylsulfat (SDS) w/w) dispergiert und mit 20 Zyklen bei 1500 bar mit einem Micron LAB 40 (APV Gaulin, Deutschland) homogenisiert. Der PCS-Durchmesser lag bei 1106 nm und der PI von 0.545 deutet auf eine sehr breite Partikelgrößenverteilung. Die Anwendung der neuen Methode (Beispiel 4 Glb-B) unter denselben Bedingungen führte zu Arzneistoffnanokristallen mit einem PCS-Durchmesser von 406 nm und einem Polydispersitätsindex von 0,264 bereits nach einem Homogenisationszyklus. Das bedeutet, das trotz der Anwendung von vielen Homogenisationszyklen mit der traditionellen Methode das Produkt außerhalb des Nanometerbereiches ist und eine breite Partikelgrößenverteilung aufweist. Die Anwendung eines einzigen Homogenisationszykluses, die gleichzeitig zu einer Halbierung der Partikelgröße führt, ist natürlich sehr produktionsgeeignet.

Der pharmazeutische Wirkstoff Hydrocortisonacetat wurde konventionell verarbeitet, in dem er in einer Tensidlösung (1% Poloxamer 188 und 0.2% SDS w/w) bei 1500 bar mit 20 Zyklen homogenisert wurde. Arzneistoffnanokristalle mit einem PCS-Durchmesser von 716 nm und einem PI von 0.45 wurden erhalten (Beispiel 7). Die Anwendung des hier beschriebenen Prozesses (Beispiel 7 Hca-B) führte zu einer Partikelgröße von 476 nm und einem PI von 0.170 bereits nach 10 Homogenisationszyklen. Wiederum wurde also eine Partikelgrößenverteilung erzielt, wie sie für parenterale Fettemulsionen beschrieben wird.

Arzneistoffnanokristalle wurden auch in Dispersionsmedien, die aus Wasser und mit Wasser mischbaren Substanzen zusammengesetzt waren, hergestellt. Wenn man den Arzneistoff Hydrocortisonacetat in einer Mischung aus Wasser:Glycerol (50:50 w/w) entsprechend der erfindungsgemäßen Methode prozessiert, erhält man nach 30 Homogenisationszyklen eine Partikelgröße von 637 nm (Beispiel 12).

Für die orale Verabreichung von ist es von besonderem Interesse, Arzneistoffnanokristalle in nichtwässrigen Medien, wie z.B. Polyethylenglykolen (PEG) oder verschiedenen natürlichen oder synthetischen Ölen und Fetten (z.B. medium chain triglycerides - MCT) herzustellen. Diese Suspensionen können dann direkt in Hard- oder Weichgelatinekapseln gefüllt werden.

Der Arzneistoff Amphotericin B wurde ebenfalls nach der erfindungsgemäßen Methode verarbeitet. Sprühgetrockneter Arzneistoff wurde dazu in einer Tensidlösung (0.5% Tween 80) in PEG 300 dispergiert und anschließend bei 1500 bar 15 Zyklen homogenisiert. Eine Partikelgröße von LD 50% 172 nm und LD 99% von 658 nm wurde erhalten (Beispiel 13).

Hochkdruckhomogenisatoren oder allgemeiner Geräte mit einer hohen Leistungsdichte unterliegen einem höheren Geräteverschleiß als Geräte mit geringerer Leistungsdichte. Trotz der Tatsache, dass Hochdruckhomogenisatoren sogar unter besonders harten Bedingungen nur zu einer unkritischen Produktkontaminations führen (Krause, K.P., et al., Heavy metal contamination of nanosuspensions produced by high-pressure homogenisation. Int J Pharm, 2000. 196(2): p. 169-72.)), ist es im Allgemeinen wünschenswert, Geräte mit niedrigerer Leistungsdichte einsetzen zu können, um Partikel im Nanometerbereich zu erzielen.

Sprühgetrocknetes Glibenclamid wurde in einer wässrigen Tensidlösung (1% Poloxamer und 0.2% SDS) dispergiert und 1 Minute bei 20.000 Umdrehungen pro Minute mit einem Ultra-Turrax (Janke & Kunkel) behandelt. Bereits dadurch konnte eine Partikelgröße von 961 nm erzielt werden (Beispiel 14)

Die Partikelgrößenbestimmung wurde unter Nutzung der Laserdiffraktometrie (LD) und der Photonenkorrelationsspektroskopie (PCS) durchgeführt. Die Laserdiffraktometrie wurde mit einem Coulter LS 230 (Beckman-Coulter, USA) durchgeführt und liefert als Ergebnis einen volumenbezogene Partikelgrößenverteilung. Die zur Bestimmung herangezogenen Parameter waren die Durchmesser 50% (LD 50%) und LD 99%. LD 50% bedeutet, dass 50% der Partikel bezogen auf deren Volumens einen Durchmesser unter dem angegebenen Wert besitzen. Die PCS-Analyse wurde mit einem Zetasizer 4 (Malvern Instruments, GB) durchgeführt. Die PCS ergibt einen mittleren Partikeldurchmesser (z-average) der Hauptpopulation und einen Polydisperitätsindex (PI) als Maß für die Breite der Partikelgrößenverteilung. Der PI für relativ enge Verteilungen liegt zwischen 0,1 - 0,2, Werte größer als 0,5 und mehr weisen auf eine sehr breite Partikelgrößenverteilung hin.

Die Differenz Scanning Kalorimetrie (DSC) wurde mit eines Mettler Toledo DSC 821 (Mettler Toledo, Deutschland). Die Aufheizrate betrug 5 K/min.

Die Erfindung ist dadurch charakterisiert, dass partikuläres Material in Nanometerbereich durch Aufwendung einer relativ geringen Anzahl an Homogenisationszyklen bzw. durch eine relativ kurze Einwirkung von Scher- und Kavitationskräften erzielt werden kann. Bereits nach 1-5 Zyklen sind die Partikeldurchmesser normalerweise unter 1000 nm, sehr oft unter 800 nm und im Falle von weicheren Materialen unter 700 nm. Eine Erhöhung der Zyklenzahl führt zu Partikeln mit einer Partikelgröße von unter 500 nm und bei höherer Zyklenzahl können 300-400 nm erreicht werden. Bei einer größeren Zyklenzahl, insbesondere bei einer Erhöhung des Homogenisationsdruckes von 1500 bar auf 2000 bar, können Größen von unter 200 nm erreicht werden, in einigen Fällen sogar Größen kleiner 100 nm.

Die Herstellung von pharmazeutischen Wirkstoffen im Nanometerbereich ist für verschiedenste Applikationswege vorteilhaft. In topischen Zubereitungen für Applikationen auf der Haut erhöhen nanokristalline Formen die Sättigungslöslichkeit, was zu einer verbesserten Penetration in die Haut führt. Nach der oralen Verabreichung ist die Auflösungsgeschwindigkeit verbessert. Die erhöhte Sättigungslöslichkeit führt zu einem erhöhten Konzentrationsgradienten, was wiederum zu erhöhten Blutkonzentrationsspiegeln führt. Auch die parenterale Verabreichung über eine intravenöse Injektion ist möglich, wobei die sich schnell auflösenden Nanokristalle die Injektion einer Lösung imitieren. Die Injektion in Körperhöhle oder die intramuskuläre Verabreichung schafft Depots, die zu verlängerten Blutspiegeln im Vergleich zur intravenösen Applikation führen.

Eine weitere Anwendung für Arzneistoffnanokristalle wäre z.B. die Verabreichung am oder im Auge, die zu einer verlängerten Verweildauer des Wirkstoffs am Auge führt. Arzneistoffnanokristalle können durch die Verwendung geeigneter Tenside oder Stabilisatoren positiv geladen werden, was zu einer erhöhten Adhäsivität an der Haut und an Hautanhangsprodukten, wie z.B. Haaren, führt. Eine Partikelgröße im Nanometerbereich ist auch z.B. für die Nahrungsmittelindustrie vorteilhaft, da Hilfsstoffe viel besser in der Nahrung verteilt werden können, z.B. Zusatzstoffe beim Backen. Nanokristalline Farbstoffe verbessern den Farbeffekt und die Farbintensität von kosmetischen Produkten, aber auch von Farben für verschiedene andere Anwendungen. Nanokristallines Material ist auch für die Textilindustrie von Interesse (z.B. um Materialien herzustellen, die eine große Adhäsivität an Textilfasern aufweisen, besonders wenn diese entgegengesetzt zur Textilfaser aufgeladen sind).

Die Erfindung beschreibt daher ganz allgemein einen Prozess, um Nanopartikel auf eine sehr effektive Weise, unter Verwendung einer Kombination aus einem schnellen Trocknungsprozess mit anschließender Anwendung von hohen Scherkräften, herzustellen. Der Wirkstoff wird in einem Lösungsmittel gelöst, Hilfsstoffe, wie Tenside, Polymere, Kohlenhydrate and andere verschiedene Hilfsstoffe können dieser Lösung zugesetzt werden. Die Hilfsstoffe können in dem verwendeten Lösemittel löslich sein oder alternativ in diesem in partikulärer Form verteilt vorliegen. Diese, den Wirkstoff in gelöster Form enthaltende Flüssigkeit, wird dann unter Verwendung von konventionellen Trocknungsgeräten (z.B. Sprühtrocknern, Wirbelschichttrockner, Trockenwalzentrockner, Dünnschichtvakuumtrockner) getrocknet oder sprühgetrocknet. Das erhaltene Pulver wird anschließend in einem flüssigen Medium, welches Wasser, ein Mischung aus Wasser mit in Wasser mischbaren Flüssigkeiten oder eine nichtwässrige Flüssigkeit sein kann, dispergiert. Das Dispersionsmedium kann z.B. Tenside, polymere Stabilisatoren oder andere Zusatzstoffe enthalten. Die entstandene Dispersion wird dann hohen Scher- und/oder Kavitationskräften unter Verwendung von Hochdruckhomogenisatoren (Jet-Stream-Homogenisatoren, Kolben-Spalt-Homogenisatoren) oder Kugelmühlen ausgesetzt. Es können auch andere Geräte Anwendung finden, die hohe Kräfte (wie z.B. Kavitation oder Ultraschall) auf die Partikel ausüben können. Die aufgewendeten Kräfte führen zu einer Verringerung der Partikelgröße in den Nanometerbereich. Die beschriebenen Methode ist dadurch charakterisiert, dass viel weniger Energie benötigt wird, als in den bisher beschriebenen Methoden zur Herstellung von Nanokristallen und dass bei der Anwendung von hohen Scher- und Kavitationskräften viel weniger Homogenisationszyklen erforderlich sind. Beide Faktoren sind vorteilhaft, da eine Produktkontamination durch Abnutzung der Geräte reduziert ist. Zusätzlich werden die Produktionskosten durch eine viel schnellere Herstellung aufgrund der verringerten Anzahl der benötigten Homogenisationszyklen reduziert. Für Wirkstoffe, deren Partikelgröße bisher kaum oder gar nicht bei Aufwendung einer akzeptablen Zyklenzahl in den Nanometerbereich gebracht werden konnte, stellt die beschriebene Methode die einzige Herstellungsmöglichkeit zur Produktion von Nanopartikeln dar.

Die erhaltenen Nanopartikel können in verschiedenen Bereichen angewendet werden, z.B. im pharmazeutischen Bereich, in der Kosmetikindustrie, der Nahrungsmittelindustrie, der Textilindustrie und andere technischen Bereichen.

Anders ausgedrückt beschreibt die vorliegende Erfindung einen Prozess zur Herstellung von pharmazeutischen, kosmetischen oder anderen Wirkstoffen in nanopartikulärer Form, d.h. die Partikel besitzen eine mittlere Größe von weniger als 1000 nm. Der Wirkstoff wird in einem Lösemittel, dem verschiedene Hilfsstoffe optional zugesetzt werden können, gelöst und anschließend schnell getrocknet. Das trockene Produkt wird in einem Dispersionmedium verteilt und die erhaltene Suspension anschließend hohen Kräften (z.B. Ultraschall, Kavitations- und/oder Scherkräften, z.B. erzeugt durch Hochdruckhomogenisatoren) ausgesetzt, was zu einer Partikelgrößenverringerung in den Nanometerbereich führt. Die erhaltenen Nanosuspensionen dienen entweder selbständig als Produkt oder werden weiterverarbeitet.

Die folgenden Beispiele sollen die Erfindung verdeutlichen.

### Beispiel 1

Es wurde nach der bekannten Methode eine Nanosuspension mit dem Wirkstoff Ibuprofen hergestellt (Charge Ibu-A). Dazu wurde 0,4g mikronisiertes Ibuprofen in 39,6g einer wässrigen Tensidlösung, die 0,08g Natriumdodecylsulfat (SDS) und 0,4g Poloxamer 188 enthielt, 1 Minute mit einem Ultra-Turrax (Janke & Kunkel, Germany) bei 9,500 Umdrehungen pro Minute dispergiert. Anschließend wurde mit einem Hochdruckhomogenisator Micron LAB 40 (APV-Homogenisers, Unna, Germany) homogenisert. Zu Beginn wurden 2 Zyklen bei 150 bar durchgeführt, anschließend 2 Zyklen bei 500 bar, dann wurde mit 1500 bar weiterhomogenisiert. Nach 40 Homogenisationszyklen bei Raumtemperatur (RT) und einem Druck von 1500 bar wurden für Charge Ibu-A mit Hilfe der Photonenkorrelationsspektroskopie (PCS) ein mittlerer Partikeldurchmesser von 1172 nm und ein Polydispersitätsindex (PI) von 0,270 erhalten. Eine weitere Erhöhung der Zyklenzahl auf 60 Zyklen führte nur zu einer weiteren nicht nennenswerten Verringerung der mittleren Partikelgröße (nach PCS) auf 1150 nm.

### Beispiel 2

Die Nanosuspension Charge Ibu-B wurde nach der erfindungsgemäßen Methode hergestellt. Dazu wurden zunächst 20g mikronisiertes Ibuprofen in 180g Ethanol (96% V/V) aufgelöst und diese Lösung mit einem Sprühtrockner Mini Spray Dryer, Modell 190 (Büchi, Switzerland) sprühgetrocknet. Die Sprühtrocknungsbedingungen waren: Volumenstrom 600 L/min, Pumpstellung 6, Aspiration 3-4, Heizrate: 3-4, Inlet-Temperatur: 72-76°C, Outlet-Temperatur: 58-60°C. Das auf diese Weise erhaltene Arzneistoffpulver wurde in der gleichen Rezepturzusammensetzung und unter identischen Bedingungen (wie unter Bsp. 1 angegeben) weiterverarbeitet. Bereits nach 20 Homogenisationszyklen bei 1500 bar wurde mittels PCS ein mittlerer Partikeldurchmesser von 636 nm bei einem PI von 0,169 erzielt. Bereits nach 5 Zyklen konnten mit PCS Partikelgrößen von 930 nm bei einem PI von 0,350 gemessen werden.

### Beispiel 3

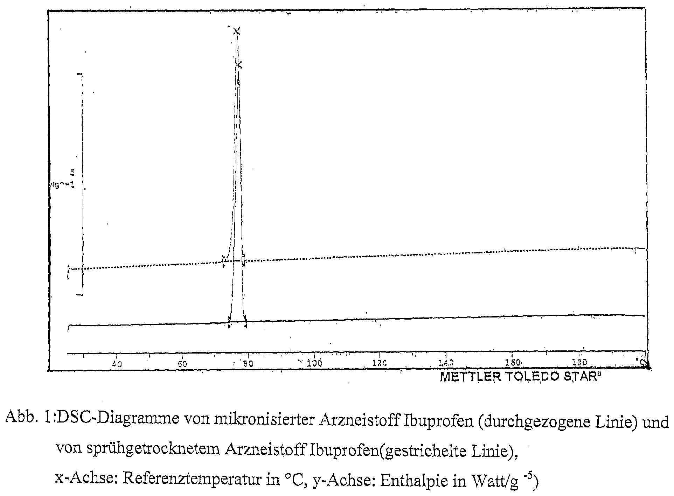

Abbildung 1 zeigt Untersuchungen des kristallinen Zustand der verwendeten Ausgangsmaterialen für die Herstellung der Charge Ibu-A (konventionell) und Charge Ibu-B (erfindungsgemäß, wie in Beispiel 2 erwähnt, hergestellt), die mit Hilfe der Differenz Scanning Calorimetry (DSC) durchgeführt wurden. Der mikronisierte Ausgansstoff besaß einen extrapolierten Schmelzpunkt von 76,60°C und eine normalisierte Schmelzenthalpie von 124,99 J/g. Das entsprechend der unter Beispiel 2 erwähnten Bedingungen hergestellte sprühgetrocknete Ibuprofen-Pulver besaß einen extrapolierten Schmelzpunkt von 76,14°C und eine normalisierte Schmelzenthalpie von 117,04 J/g. Anhand dieses Beispiels lässt sich feststellen, dass die Ausgangsmaterialien der Charge A und B sich nicht hinsichtlich ihrer Kristallinität unterscheiden, so dass die verbesserte Effektivität bei der Homogenisierung nicht auf eine Abnahme der Kristallinität zurückzuführen ist.

### Beispiel 4

Für Beispiel 4 wurden zum Zwecke des Vergleichs zwei Nanosuspensionen mit dem Wirkstoff Glibenclamid hergestellt. Charge Glb-A wurde nach der bekannten Methode hergestellt (Ansatzgröße 40g, 0,4g Glibenclamid, 0,08g SDS, 0,4g Poloxamer 188, 39,12g Wasser; Herstellungsbedingungen wie unter Beispiel 1 angegeben), Charge Glb-B nach der erfindungsgemäßen Methode. Dafür wurden 5g Glibenclamid zusammen mit 1g SDS in 794g Ethanol (96% V/V) gelöst und mit einem Sprühtrockner Mini Spray Dryer, Modell 190 (Büchi, Switzerland) sprühgetrocknet. Die Sprühtrocknungsbedingungen waren: Volumenstrom 600 L/min, Pumpstellung 10, Aspiration 4, Heizrate: 4-5, Inlet-Temperatur: 90°C, Outlet-Temperatur: 58-60°C. Für die Herstellung der Wirkstoffdispersion wurden 480 mg des sprühgetrockneten Pulvers in 39,52g einer wässrigen Lösung, die 0,4g Poloxamer 188 enthielt, entsprechend der unter Beispiel 1 angegebenen Methode dispergiert und anschließend, wie ebenfalls in Beispiel 1 angegeben, mittels eines Micron LAB 40 hochdruckhomogenisiert. Für Charge Glb-A wurde nach einem Homogenisationszyklus bei 1500 bar mit PCS eine mittlere Partikelgröße von 1106 nm bei einem PI von 0.545 erhalten. Im Unterschied dazu zeigte Charge Glb-B bereits nach dem ersten Homogenisationszyklus eine Größe von 506 nm bei einem PI von 0,385. Es zeigt sich also, dass bei der erfindungsmäßigen Methode ein einziger Homogenisationsschritt ausreicht, um Partikel mit einem mittleren Durchmesser im Nanometer-Bereich zu erhalten. Des Weiteren zeigen die nach der neuen Methode erhaltenen Suspensionen eine homogenere Patikelgrößenverteilung.

### Beispiel 5

Anhand der für Beispiel 4 hergestellten Suspensionen wurde untersucht, welchen Einfluss die erfinderische Methode auf die benötigte Anzahl der Zyklen zum Erreichen der Grenzdispersität besitzt. Während für Charge Glb-A nach der bekannten Methode 40 Homogensiationszyklen erforderlich waren, um eine mittlere Partikelgröße von 500 nm bei einem PI von 0,25 zu erhalten, waren für die erfindungsgemäß hergestellte Charge Glb-B bereits 15 Zyklen ausreichend, um Partikelgrößen von 431 nm bei einem PI von 0,250. 20 Zyklen ergaben im Fall von Glb-B einen mittleren Partikeldurchmesser von 406 nm bei einem PI von 0,264. Dadurch ergibt sich eine entscheidend gesteigerte Effektivität bei der Produktion von Nanosuspensionen, da einerseits die erforderliche Zyklenzahl deutlich reduziert werden kann und andererseits die eingebrachte Leistungsdichte zu deutlich kleineren Partikeln führt.

### Beispiel 6

Um einen Unterschied in der Teilchengröße des Ausgangsmaterials als Ursache für die deutlich besseren Ergebnisse hinsichtlich der Partikelgröße von Charge Glb-B aus Beispiel 5 auszuschließen, wurde die volumenbasierte Partikelgrößenverteilung beider Ausgangsmaterialien für den Hochdruckhomogenisationsprozess mit Hilfe der Laserdiffraktometrie bestimmt. Dafür wurden die Pulver jeweils in einer wässrigen SDS-Lösung (0,05% SDS) durch Rühren dispergiert und anschließend als Suspension mit einem Coulter LS 230 vermessen. Abbildung 2 zeigt, dass kein nennenswerter Unterschied zwischen normal mikronisiertem Ausgangsmaterial und dem erfindungsgemäß durch Sprühtrocknung hergestelltem Pulver (entsprechend Beispiel 4) besteht, so dass eine veränderte Größe des Ausgangsmaterials als maßgebliche Ursache für die verbesserte Effektivität ausgeschlossen werden kann.

### Beispiel 7

Es wurden zwei Nanosuspensionen mit dem Wirkstoff Hydrocortisonacetat hergestellt. Charge Hca-A wurde wie unter Beispiel 1 (gleiche Rezeptur und Produktionsbedingungen) angegeben nach der bekannten Methode hergestellt, Charge Hca-B nach der erfindungsgemäßen Methode. Dafür wurden 3g Hydrocortisonacetat und 3g Poloxamer 188 in 794g Ethanol 96% (V/V) gelöst und anschließend mit einem Sprühtrockner Mini Spray Dryer, Modell 190 (Büchi, Switzerland) sprühgetrocknet. Die Sprühtrocknungsbedingungen waren: Volumenstrom 600 L/min, Pumpstellung 12, Aspiration 3-4, Heizrate: 3-4, Inlet-Temperatur: 90°C, Outlet-Temperatur: 58-60°C. Das so erhaltene Pulver wurde dann für die Herstellung von Charge Hca-B weiterverwendet. Für Charge Hca-A wurde nach 20 Homogenisationszyklen eine mittlere Partikelgröße von 716 nm bei einem PI von 0,450 erhalten. Charge Hca-B wurde prinzipiell auf die gleiche Weise wie Charge Hca-A hergestellt, es wurde jedoch 800 mg des sprühgetrockneten Pulvers in 40g einer wässrigen Lösung von 0.08g SDS dispergiert und dann entsprechend der unter Beispiel 1 erwähnten Bedingungen für den Homogenisationsprozess weiterverfahren. Im Unterschied zur Charge Hca-A zeigte die Charge Hca-B bereits nach 10 Homogenisationszyklen eine Größe von 476 nm bei einem PI von 0,170. Es zeigt sich also, dass bei der erfindungsmäßigen Methode wenige Homogenisationsschritte ausreichen, um Nanosuspensionen zu erhalten, die eine Partikelgrößenverteilung im Bereich parenteraler Fettemulsionen aufweisen.

### Beispiel 8

Erfindungsgemäß sprühgetrocknetes Ibuprofen, hergestellt wie in Beispiel 2 erwähnt, besitzt in der mikroskopischen Abbildung mit 1000-facher Vergrößerung eine kristalline Erscheinung und eine Partikelgrößenverteilung von ca. 1-5*µ*m. Makroskopisch ist es ein weißes, lockeres Pulver mit ebenfalls kristallinem Erscheinungsbild.

### Beispiel 9

Erfindungsgemäß sprühgetrocknetes Ibuprofen, hergestellt wie in Beispiel 2 erwähnt jedoch mit Zusatz von 0.2% SDS zur Lösung, besitzt in der mikroskopischen Abbildung mit 1000-facher Vergrößerung eine kristalline Erscheinung und eine Partikelgrößenverteilung von ca. 1-5*µ*m. Die Partikel sind insgesamt etwas kleiner, als im Beispiel ohne Tensidzusatz. Makroskopisch ist es ein weißes, lockeres Pulver mit ebenfalls kristallinem Erscheinungsbild.

### Beispiel 10

Erfindungsgemäß sprühgetrocknetes Glibenclamid, hergestellt wie in Beispiel 4 erwähnt, jedoch ohne Tensidzusatz im Lösungsmittel, besitzt in der mikroskopischen Abbildung mit 1000-facher Vergrößerung eine sehr geordnete, gleichmäßig runde Erscheinung und eine Partikelgrößenverteilung von ca. 2-4*µ*m. Makroskopisch ist es ein weißes Pulver mit starker elektrostatischer Aufladung.

### Beispiel 11

Erfindungsgemäß sprühgetrocknetes Glibenclamid, hergestellt wie in Beispiel 4 erwähnt besitzt in der mikroskopischen Abbildung mit 1000-flacher Vergrößerung ebenfalls eine sehr geordnete, gleichmäßig runde Erscheinung und eine Partikelgrößenverteilung von ca. 2-4*µ*m. Makroskopisch ist es ebenfalls ein weißes Pulver mit starker elektrostatischer Aufladung.

### Beispiel 12

Hydrocortisonacetat wurde in einer Mischung aus Wasser:Glycerol (50:50 w/w) entsprechend der erfindungsgemäßen Methode behandelt. Dazu wurde ein sprühgetrocknetes Arzneistoffpulver entsprechend Beispiel 7 Charge Hca-B hergestellt, 0,8g dieses Pulvers wurden in 39,2g einer Wasser:Glycerol-Mischung (50:50 w/w) dispergiert und entsprechend der unter Beispiel 1 angegebenen Methode einem Hochdruckhomogenisationsprozess unterzogen. Nach 30 Homogenisationszyklen betrug die mittlere Partikelgröße bestimmt mit PCS 637 nm.

### Beispiel 13

Der Arzneistoff Amphotericin B wurde ebenfalls nach der erfindungsgemäßen Methode verarbeitet. Sprühgetrocknetes Arzneistoffpulver wurde hergestellt, in dem 0.2g Amphotericin B in 10 g Dimethylsulfoxid (DMSO) gelöst wurden, diese Lösung in 189,8g Methanol gegeben wurde und diese Lösung anschließend mit einem Sprühtrockner Mini Spray Dryer, Modell 190 (Büchi, Switzerland) sprühgetrocknet wurde. Die Sprühtrocknungsbedingungen waren: Volumenstrom 600 L/min, Pumpstellung 10, Aspiration 3, Heizrate: 1, Inlet-Temperatur: 56°C, Outlet-Temperatur: 34°C. Das auf diese Weise erhaltene Pulver wurde in PEG 300 unter Zusatz von 0,5% Tween 80 dispergiert und anschließend bei 1500 bar mit einem Micron LAB 40 (APV-Homogenisers, Unna, Germany), wie unter Beispiel 1 erwähnt, homogenisiert. Die Partikelgrößen bestimmt durch Laserdiffraktometrie betrugen 172 nm (LD 50%) und 658 nm (LD99%).

### Beispiel 14

Sprühgetrocknetes Glibenclamid (aus Beispiel 4) wurde in einer wässrigen Tensidlösung (1% Poloxamer) dispergiert und 1 Minute bei 20,000 Umdrehungen pro Minute mit einem Ultra-Turrax mit einem Vorsatz T 25 (Janke & Kunkel, Germany) behandelt. Bereits dadurch konnte eine mit PCS gemessene Partikelgröße von 961 nm erzielt werden.

Ein schwerlöslicher Stoff im Sinne der Erfindung besitzt in dem jeweiligen Lösungsmittel/Dispersionsmittel, bezogen auf das Gewicht des Lösungsmittels/Dispergiermittels, eine maximale Löslichkeit von 1 %, bevorzugt kleiner 0,1 % uns insbesondere kleiner als 0,01 %.

## Patentansprüche

1. Verfahren zur Herstellung ultrafeiner Suspensionen, **dadurch gekennzeichnet, daß**
- eine bei 20°C als Feststoff vorliegende Substanz in einem Lösungsmittel gelöst wird,
- dem sich im Trocknungsvorgang befindlichen Anteil dieser Lösung sehr schnell innerhalb von 5 Sekunden das Lösungsmittel im Wesentlichen entzogen wird,
- das dabei erhaltene Pulver, dessen Partikel eine durchschnittliche Partikelgröße im Bereich von 1 bis 50 µm (gemäß Laserdiffraktometrie) aufweisen, in einem Dispersionsmedium dispergiert wird und
die so erhaltene Suspension unter Anwendung von hohen Kräften 10⁹ bis 10¹³ W/m³ zu einer Suspension weiterverarbeitet wird, deren Feststoffpartikel eine durchschnittliche Partikelgröße (gemäß Photonenkorrelationsspektroskopie PCS) unter 1 µm (1000 nm), und insbesondere unter 400 nm, speziell unterhalb von 200 nm aufweisen.

2. Verfahren nach Anspruch 1, **dadurch gekennzeichnet, daß** die hohen Kräfte Scher-, Kavitations-, Mahl- und/oder Ultraschallkräfte sind und insbesondere Hochdruckhomogenisatoren, Jet-Stream-Geräte, Kugelmühlen, Hochscherungsmischer oder Ultraschallapparaturen eingesetzt werden, wobei das eingesetzte Gerät vorzugsweise mit einer Leistungsdichte von im Bereich von 10⁹ oder 10¹⁰ bis 10¹² oder 10¹³ W/m³ arbeitet.

3. Verfahren nach Anspruch 1 oder 2, **dadurch gekennzeichnet, daß** die für die Auflösung eingesetzten Lösungsmittel Wasser, Mischungen von Wasser und mit Wasser vollständig oder teilweise mischbaren Flüssigkeiten oder hydrophile Flüssigkeiten, insbesondere Alkohole, bevorzugt Methanol, Ethanol oder Isopropanol oder andere organische Lösungsmittel, oder mit Wasser nicht mischbare Flüssigkeiten, insbesondere Chloroform, Dimethylsulfoxid oder Dichlormethan sind, wobei bevorzugte Lösungsmittel Wasser, N-Methyl-2-pyrrolidinon, 2-Pyrrolidon, Dimethylacetamid, Ethanol, Isopropanol, Aceton, Chloroform, Dichlormethan, Dimethylsulfoxid, N-Propanol, Glycerol, Ethylenglycol, Dimethylformamid, Dimethylacetamid oder eine organische Säure, insbesondere Essigsäure, Ameisensäure, Milchsäure oder Fumarsäure, sind, wobei gegebenenfalls eine Mischung aus zwei oder mehr derselben eingesetzt wird.

4. Verfahren nach Anspruch 1, 2 oder 3, **dadurch gekennzeichnet, daß** die zuerst herzustellende Lösung oder die Suspension mit den zu zerkleinernden Partikeln außerdem einen oder mehrere weitere Hilfsstoffe und dispersionsstabilisierende Substanzen enthält, insbesondere Tenside, Stabilisatoren, insbesondere vom Typ der Antiflokkulantien und Polymere, sowie inerte Füllstoffe, wobei die Konzentrationen solche Komponenten pro Komponente, bezogen auf das Gesamtgewicht der Lösung, bevorzugt im Bereich von 1 bis 90%, insbesondere von 1 bis 20% und bevorzugt unterhalb von 10% liegen, idealerweise unterhalb von 0,01 bis 5% liegen.

5. Verfahren nach Anspruch 4, **dadurch gekennzeichnet, daß** die stabilisierenden Substanzen Verbindungen aus der Reihe der Poloxamere, Poloxamine, ethoxylierten Mono- und Diglyceride, ethoxylierten Lipide und Lipoide, ethoxylierten Fettalkohole und Alkylphenole, ethoxylierten Fettsäureester, Polyglycerinether und -ester, Lecithine, Ester und Ether von Zuckern oder Zuckeralkoholen mit Fettsäuren oder Fettalkoholen, Phospholipide und Sphingolipide, Sterine, deren Ester oder Ether sowie Mischungen dieser Verbindungen umfassen.

6. Verfahren nach Anspruch 4, **dadurch gekennzeichnet, daß** die stabilisierenden Substanzen Eilecithin, Sojalecithin oder hydriertes Lecithin, deren Mischungen oder Mischungen aus einem oder beiden Lecithinen mit einer oder mehreren Phopholipidkomponenten, Cholesterin, Cholesterinpalmitat, Stigmasterin, andere Sterine oder Mischungen derselben umfassen.

7. Verfahren nach Anspruch 4, **dadurch gekennzeichnet, daß** die Stabilisatoren Diacetylphosphat, Phosphatidylglycerol, gesättigte oder ungesättigte Fettsäuren, Natriumcholat, Peptisatoren, Aminosäuren oder Mischungen derselben umfassen.

8. Verfahren nach einem der Ansprüche 4 bis 7, **dadurch gekennzeichnet, daß** viskositätserhöhende Stoffe, insbesondere Celluloseether und -ester, Polyvinylderivate, Alginate, Xanthane, Pektine, Polyacrylate, Poloxamere oder Poloxamine, Polyvinlyalkohol, Polyvinylpyrrolidon oder Mischungen derselben enthalten sind.

9. Verfahren nach einem der Ansprüche 4 bis 8, **dadurch gekennzeichnet, daß** die Lösung außerdem Hilfsstoffe, wie Zucker oder Zuckeralkohole, insbesondere Glucose, Mannose, Trehalose, Mannit und Sorbit, Fructose, Natriumcitrat, Natriumhydrogenphosphat, Natriumdihydrogenphosphat, Natriumchlorid, Kaliumchlorid, Calciumchlorid, Glycerin, Farbstoffe oder Pigment enthalten sind.

10. Verfahren einem der Ansprüche 1 bis 9, **dadurch gekennzeichnet, daß** der Lösungsmittelentzug/die Trocknung mit einem Sprühtrockner durchgeführt wird.

11. Verfahren nach einem der Ansprüche 1 bis 9, **dadurch gekennzeichnet, daß** der Lösungsmittelentzug/die Trocknung mit einem Wirbelschichttrockner, Trockenwalzentrockner, Dünnschichtvakuumtrockner oder anderen Trocknern oder Trocknungsmethoden, die zu einem schnellen Entzug des Lösungsmittels führen, durchgeführt wird.

12. Verfahren nach einem der Ansprüche 1 bis 11, **dadurch gekennzeichnet, daß** der Lösungsmittelentzug/die Trocknung des in dem Trocknungsvorgang befindlichen Anteil der hergestellten Lösung innerhalb von 2, insbesondere von 1, bevorzugter 0,5, und noch bevorzugter innerhalb von 0,1 Sekunden erfolgt.

13. Verfahren nach einem der Ansprüche 1 bis 12, **dadurch gekennzeichnet, daß** der Lösungsmittelentzug/die Trocknung zu einem Restlösungsmittelgehalt/Restfeuchtigkeit von maximal 10 Gew.%, bevorzugt 5 Gew.%, bevorzugter 1 Gew.%, insbesondere 0,5 Gew.% spezieller 0,1 Gew.% oder darunter führt.

13. Verfahren nach einem der Ansprüche 1 bis 13, **dadurch gekennzeichnet, daß** das nach dem Entzug des Lösungsmittels erhaltene Pulver kristallin, teilkristallin oder amorph ist.

14. Verfahren nach einem der Ansprüche 1 bis 14, **dadurch gekennzeichnet, daß** das nach dem Entzug des Lösungsmittels erhaltene durch Rühren mit Blattrührern, Rotor-Stator-Systemen, statischen Mischern, optional zusätzlicher Passage durch eine Rotor-Stator-Kolloidmühle, Pulver in dem Dispersionsmedium dispergiert wird, so daß eine Suspension erhalten wird.

15. Verfahren nach Anspruch 15, **dadurch gekennzeichnet, daß** als Dispersionsmedium Wasser, Mischungen aus Wasser und mit Wasser mischbaren Flüssigkeiten, nicht-wäßrige Medien oder organische Lösungsmittel oder lipophile Flüssigkeiten, insbesondere Öle und fette Öle eingesetzt werden.

16. Verfahren nach einem der Ansprüche 1 bis 16, **dadurch gekennzeichnet, daß** der zu lösende Feststoff ein Arzneimittelwirkstoff, ein kosmetischer Wirkstoff, ein Zusatzstoff für Nahrungsmittel, ein Farbstoff oder ein Pigment ist.

17. Verfahren nach einem der Ansprüche 1 bis 17, **dadurch gekennzeichnet, daß** die zur Zerkleinerung der Partikel der Suspension einwirkende Kraft durch einen Hochdruckprozess aufgewendet wird, wobei insbesondere Homogenisatoren vom Kolben-Spalt-Typ (z.B. APV Gaulin, NiroSoavi, Avestin), vom Typ des "Jet Streams" (z.B. Microfluidizer) oder eine French Press (SLM Instruments, Urbana, Ill., USA) eingesetzt werden.

18. Verfahren nach einem der Ansprüche 1 bis 18, **dadurch gekennzeichnet, daß** bei Verwendung von Hochdruckhomogenisatoren der Homogenisationsdruck oberhalb 100 bar liegt, bevorzugt bei oder oberhalb 500 bar, insbesondere bei oder oberhalb 1500 bar und am bevorzugtesten bei oder oberhalb 2000 bar liegt.

19. Verfahren nach einem der Ansprüche 18 oder 19, **dadurch gekennzeichnet, daß** bei Verwendung von Hochdruckhomogenisatoren zur Erreichung der mittleren PCS-Partikelgröße unterhalb von 1 µm die Zahl der Homogenisationszyklen weniger als 10 insbesondere weniger als 5, bevorzugt weniger als 3 und speziell nur 1 beträgt.

20. Verfahren nach einem der Ansprüche 1 bis 20, **dadurch gekennzeichnet, daß** die nach der Partikelzerkleinerung erhaltene Partikelsuspension entweder direkt oder nach Abtrennung der Partikel zum Einsatz kommt, insbesondere bei pharmazeutischen und kosmetischen Applikationen, vorzugsweise in Form von Tabletten und Kapseln, Cremes, Salben oder Pulvern zur Rekonstitution vor der Anwendung.

21. Verfahren nach einem der Ansprüche 1 bis 21, **dadurch gekennzeichnet, daß** die erhaltene Suspension zu Zwischen-oder Endprodukten weiterverarbeitet wird.

22. Verfahren nach einem der Ansprüche 1 bis 22, **dadurch gekennzeichnet, daß** die erhaltene Suspension durch Aufbringen auf Zuckerpellets oder durch Einarbeitung in Matrixpellets weiterverarbeitet wird.

23. Verfahren nach einem der Ansprüche 1 bis 22, **dadurch gekennzeichnet, daß** die erhaltene Suspension sprühgetrocknet oder lyophillisiert wird.

24. Verfahren nach einem der Ansprüche 1 bis 22, **dadurch gekennzeichnet, daß** die erhaltene Suspension als Granulierungsflüssigkeit eingesetzt wird und durch einen Granulierungsschritt ein Granulat erhalten wird, das direkt oder nach Verpressung zu Tabletten appliziert wird.

25. Verfahren nach einem der Ansprüche 1 bis 22, **dadurch gekennzeichnet, daß** die erhaltene Suspension direkt oder nach Modifikation in Hart- oder Weichgelatinekapseln gefüllt wird.

26. Verfahren nach einem der Ansprüche 1 bis 26, **dadurch gekennzeichnet, daß** der Polydispersitätsindex (PI) kleiner als 0,35, bevorzugt kleiner 0,30, insbesondere kleiner als 0,2 ist und bevorzugt im Bereich von < 0,2 bis 0,1 liegt.

## Claims

1. Method for the production of ultra-fine suspensions, **characterized in that**
- a substance, which is a solid at 20 °C, is dissolved in a solvent,
- the solvent is very rapidly and substantially removed from the part of the obtained solution, which is present in the drying process, within 5 seconds,
- the powder obtained thereby, the particles of which have an average particle size in the range of 1 to 50 µm (according to laser diffractometry), is dispersed in a dispersing medium, and
the thus obtained suspension is further processed, by applying high forces of 10⁹ to 10¹³ W/m³, into a suspension the solid particles of which having an average particle size (according to photon correlation spectroscopy PCS) below below 1 µm (1000 nm), and particularly preferred below 400 nm, especially below 200 nm.

2. Method of claim 1 **characterized in that** the high forces are shear forces, cavitation forces, milling forces and/or ultrasonic forces, and in particular high pressure homogenisators, jet stream devices, ball mills, high shear mixers or ultrasonic devices are used, wherein the device used works with a power density in the range of 10⁹ or 10¹⁰ to 10¹² or 10¹³ W/m³.

3. Method of claim 1 or 2, **characterized in that** the solvents used for dissolution are water, mixtures of water with completely water miscible or partially water miscible liquids or hydrophilic liquids, in particular alcohols, preferably methanol, ethanol or isopropanol, or other organic solvents, or with water immiscible liquids, in particular chloroform, dimethylsulfoxide or dichloromethane, preferred solvents being water, N-methyl-2-pyrrolidone, 2--pyrrolidone, dimethyl acetamide, ethanol, isopropanol, acetone, chloroform, dichloromethane, dimethyl sulfoxide, n-propanol, glyerol, ethylene glycol, dimethyl formamide, dimethyl acetamide or an organic acid, in particular acetic acid, formic acid, lactic acid or fumaric acid, wherein optionally mixtures of two or more thereof are used.

4. Method according to claim 1, 2 or 3, **characterized in that** the solution to be produced first or the suspension containing the particles to be comminuted also contains one or more further auxiliary substances and dispersion stabilizing substances, in particular surfactants, stabilizers, in particular of the type of anti-flocculants and polymers, as well as inert fillers, wherein the concentration of such components per components is, based on the total weight of the solution, in the range of 1 to 90%, in particular 1 to 20% and preferably below 10%, ideally below 0.01 to 5%.

5. Method according to claim 4, **characterized in that** the stabilizing substances comprise compounds of the group of poloxamers, poloxamines, ethoxylated mono- and diglycerides, ethoxylated lipids and lipoids, ethoxylated fatty alcohols and alkyl phenols, ethoxylated fatt acid esters, polyglycerinethers and -esters, lecithines, esters and ethers of sugars or sugar alcohols with fatty acids or fatty alcohols, phospholipides and sphingolipides, sterines, their esters or ethers, as well as mixtures of such compounds.

6. Method according to claim 4, **characterized in that** the stabilizing substances comprise egg lecithine, soy lecithine or hydrogenated lecithine, their mixtures or mixtures of one or both lecithines with one or more phospholipde components, cholesterine, cholesterine palmitate, stigmasterine, other sterines, or mixtures thereof.

7. Method according to claim 4, **characterized in that** the stabilizers comprise diacethyl phosphate, phosphatidyl glycerol, saturated or unsaturated fatty acids, sodium cholate, peptization agents, amino acids, or mixtures thereof.

8. Method according to any of claims 4 to 7, **characterized in that** viscosity increasing substances are present, in particular cellulose ethers and esters, polyvinyl derivatives, alginates, xanthanes, pectines, polyacrylates, poloxamers or poloxamines, polyvinyl alcohol, polyvinyl pyrrolidone, or mixtures thereof.

9. Method according to any of claims 4 to 8, **characterized in that** the solution also contains auxiliary substances such as sugars or sugar alcohols, in particular glucose, mannose, trehalose, mannitol and sorbitol, fructose, sodium citrate, sodium hydrogen phosphate, sodium dihydrogen phosphate, sodium chloride, potassium chloride, calcium chloride, glycerin, dyes or pigments.

10. Method according to any of claims 1 to 9, **characterized in that** the solvent removal/the drying is effected by use of a spray dryer.

11. Method according to any of claims 1 to 9, **characterized in that** the solvent removal/the drying is effected by use of fluidized bed dryer, dry roller dryer, thin layer vacuum dryer, or other dryers or drying methods, which result in a rapid removal of solvent.

12. Method according to any of claims 1 to 11, **characterized in that** the solvent removal/the drying of the part of the obtained solution, which is present in the drying process, occurs within 2 seconds, in particular 1 second, more preferred 0.5 second and still more preferred within 0.1 second.

13. Method according to any of claims 1 to 12, **characterized in that** the solvent removal/the drying results in a residual solvent content/residual humidity of at maximum 10 wt.%, preferably 5 wt.%, more preferred 1 wt.%, in particular 0.5 wt.%, especially 0.1 wt.% or below.

14. Method according to any of claims 1 to 13, **characterized in that** the powder obtained after the solvent removal is crystalline, partially crystalline or amorphous.

15. Method according to any of claims 1 to 14, **characterized in that** the powder obtained after the solvent removal is dispersed in a dispersing agent by way of stirring using blade stirrers, rotor-stator systems, static mixers, optionally additional passage through a rotor-stator colloid mill, so that a suspension is obtained.

16. Method according to claim 15, **characterized in that** as dispersing medium water, mixtures of water with water miscible liquids, non-aqueous media, or organic solvents or lipophilic liquids, in particular oils and fatty oils are used.

17. Method according to any of claims 1 to 16, **characterized in that** the solid to be dissolved is a medical drug, a cosmetically active agent, an additive for food, a dye or a pigment.

18. Method according to any of claims 1 to 17, **characterized in that** the force acting on the suspension for comminution of the particles is applied by way of a high pressure process, wherein in particular homogenizers of the piston-gap homegenizer type (e.g. APV Gaulin, NiroSoavi, Avestin), the "jet stream" type (e.g. Microfluidizer), or the French press type (SLM Instruments, Urbana, Ill. USA) are used.

19. Method according to any of claims 1 to 18, **characterized in that** when using high pressure homogenizers the homogenization pressure is above 100 bar, in particular at or above 500 bar, particularly at or above 1500 bar and most preferred at or above 2000 bar.

20. Method according to any of claims 18 or 19, **characterized in that** when applying high pressure homogenizers for obtaining an average PCS particle size below 1 µm the number of homogenization cycles is below 10, in particular below 5, preferably below 3 and especially is only 1.

21. Method according to any of claims 1 to 20, **characterized in that** the particle suspension obtained after said particle comminution is used either directly or after separation of the particles, in particular in pharmaceutical and cosmetic applications, preferably in form of tablets and capsules, cremes, oinments, or powders for reconstruction prior to the application.

22. Method according to any of claims 1 to 21, **characterized in that** the obtained suspension is further processed to intermediate products or final products.

23. Method according to any of claims 1 to 22, **characterized in that** the obtained suspension is further processed by way of application onto sugar pellets or by way of incorporation into matrix pellets.

24. Method according to any of claims 1 to 22, **characterized in that** the obtained suspension is spray dried or lyophilized.

25. Method according to any of claims 1 to 22, **characterized in that** the obtained suspension is used as granulating liquid, and by way of a granulation step a granulate is obtained which is applied directly or after compaction into tablets.

26. Method according to any of claims 1 to 22, **characterized in that** the obtained suspension is directly or after modification filled into hard or soft gelatin capsules.

27. Method according to any of claims 1 to 26, **characterized in that** the polydispersity index (PI) is below 0.35, preferably below 0.30, in particular below 0.2 and is preferably within the range of < 0.2 to 0.1.

## Revendications

1. Procédé de production de suspensions ultrafines, **caractérisé en ce que**
- une substance présente sous forme d'une matière solide à 20 °C est dissoute dans un solvant,
- le solvant est sensiblement éliminé de la partie, se trouvant dans le processus de séchage, de cette solution très rapidement en secondes,
- la poudre ainsi obtenue, dont les particules ont une taille de particule moyenne dans la gamme de 1 à 50 µm sont (mesurée par diffractométrie laser), est dispersée dans un milieu de dispersion et
la suspension ainsi obtenue est ensuite traitée à l'aide de fortes forces de 10⁹ à 10¹³ W/m³ pour donner une suspension dont les particules de matières solides ont une taille moyenne de particules (mesurée par spectroscopie de corrélation de photons, PCS) de moins de 1 *µ*m (1000 nm), et en particulier de moins de 400 nm, en particulier de moins de 200 nm.

2. Procédé selon la revendication 1, **caractérisé en ce que** les fortes forces sont des forces de cisaillement, de cavitation, de broyage et/ou d'ultrasons, et **en ce que** l'on utilise en particulier des homogénéisateurs à haute pression, des dispositifs à Jet et flux, des broyeurs à boulets, un mélangeur à fort cisaillement ou des appareils à ultrasons, l'appareil utilisé fonctionnant de préférence à une puissance volumique dans la gamme de 10⁹ ou 10¹⁰ à 10¹² ou 10¹³ W/m³.

3. Procédé selon la revendication 1 ou 2, **caractérisé en ce que** les solvants utilisés pour la dissolution est l'eau, des mélanges d'eau et de liquides totalement ou partiellement miscibles à l'eau ou des liquides hydrophiles tels que des alcools, de préférence le méthanol, l'éthanol ou l'isopropanol, ou d'autres solvants organiques ou des liquides non miscibles à l'eau, en particulier le chloroforme, le diméthylsulfoxyde ou le dichlorométhane, les solvants préférés étant l'eau, la N-méthyl-2-pyrrolidinone, la 2-pyrrolidone, le diméthylacétamide, l'éthanol, l'isopropanol, l'acétone, le chloroforme, le dichlorométhane, le diméthylsulfoxyde, le n-propanol, le glycérol, l'éthylène-glycol, le diméthylformamide, le diméthylacétamide ou un acide organique, en particulier l'acide acétique, l'acide formique, l'acide lactique ou l'acide fumarique, un mélange de deux de ceux-ci ou plus étant éventuellement utilisé.

4. Procédé selon la revendication 1, 2 ou 3, **caractérisé en ce que** la première solution à préparer ou la suspension pourvue des particules à broyer contient également une ou plusieurs autres substances secondaires et des substances de stabilisation de dispersion, en particulier des tensioactifs, des stabilisants, notamment du type des anti-floculants et des polymères, ainsi que des charges inertes, les concentrations de ces composants pour chaque composant, sur la base du poids total de la solution, étant de préférence dans la gamme de 1 à 90%, en particulier de 1 à 20% et de préférence de moins de 10%, typiquement de moins de 0,01 à 5%.

5. Procédé selon la revendication 4, **caractérisé en ce que** les substances de stabilisation comportent des composés de la série des poloxamères, poloxamines, éthoxylésmono- et diglycérides, lipides et lipoïdes éthoxylés, alcools gras et alkylphénols éthoxylés, esters d'acides gras éthoxylés, éthers et esters de polyglycérol, lécithines, esters et éthers de sucres ou d'alcools de sucre avec des acides gras ou des alcools gras, phospholipides et sphingolipides, stérols, leurs esters ou éthers et mélanges de ces composés.

6. Procédé selon la revendication 4, **caractérisé en ce que** les substances de stabilisation comportent la lécithine d'oeuf, la lécithine de soja ou la lécithine hydrogénée, leurs mélanges ou des mélanges d'une des lécithines, ou des deux, avec un ou plusieurs composants phopholipidiques, le cholestérol, le palmitate de cholestérol, le stigmastérol, d'autres stérols, ou leurs mélanges.

7. Procédé selon la revendication 4, **caractérisé en ce que** les stabilisants comportent le diacétylphosphate, le phosphatidylglycérol, les acides gras saturés ou insaturés, le cholate de sodium, les peptisants, les acides aminés ou leurs mélanges.

8. Procédé selon l'une des revendications 4 à 7, **caractérisé en ce que** des substances augmentant la viscosité sont présentes, en particulier les éthers et esters de cellulose, les dérivés polyvinyliques, les alginates, les xanthanes, les pectines, les polyacrylates, les poloxamères ou les poloxamines, l'alcool polyvinylique, la polyvinylpyrrolidone ou leurs mélanges.

9. Procédé selon l'une des revendications 4 à 8, **caractérisé en ce que** la solution contient en outre des substances secondaires, telles que des sucres ou des alcools de sucre, en particulier le glucose, le mannose, le tréhalose, le mannitol et le sorbitol, le fructose, le citrate de sodium, l'hydrogénophosphate de sodium, le dihydrogénophosphate de sodium, le chlorure de sodium, le chlorure de potassium, le chlorure de calcium, le glycérol, des colorants ou des pigments.

10. Procédé selon l'une des revendications 1 à 9, **caractérisé en ce que** l'élimination de solvant/le séchage est effectué avec un séchoir à pulvérisation.

11. Procédé selon l'une des revendications 1 à 9, **caractérisé en ce que** l'élimination de solvant/le séchage est effectué avec un séchoir à lit fluidisé, un séchoir à tambour sec, un séchoir sous vide à film mince ou d'autres séchoirs ou procédés de séchage qui conduisent à une élimination rapide du solvant.

12. Procédé selon l'une des revendications 1 à 11, **caractérisé en ce que** l'élimination de solvant/le séchage de la partie, se trouvant dans le processus de séchage, de la solution préparée est effectué en 2 secondes, notamment en 1 seconde, plus préférablement en 0,5 seconde, et encire plus préférablement en 0,1 seconde.

13. Procédé selon l'une des revendications 1 à 12, **caractérisé en ce que** l'élimination de solvant/le séchage donne une teneur résiduelle en solvant/une humidité résiduelle de 10% en poids, de préférence 5% en poids, plus préférablement de 1% en poids, en particulier de 0,5% en poids, plus particulièrement 0,1% en poids maximum.

14. Procédé selon l'une des revendications 1 à 13, **caractérisé en ce que** la poudre obtenue après élimination du solvant est cristalline, partiellement cristalline ou amorphe.

15. Procédé selon l'une des revendications 1 à 14, **caractérisé en ce que** la poudre obtenue après élimination du solvant par agitation avec des agitateurs à lames, des systèmes à rotor et stator, des mélangeurs statiques, par passage supplémentaire optionnelle dans un broyeur colloïdale à rotor et stator, est dispersée dans le milieu de dispersion de façon à obtenir une suspension.

16. Procédé selon la revendication 15, **caractérisé en ce que** on utilise comme milieu de dispersion de l'eau, des mélanges d'eau et de liquides miscibles à l'eau, des milieux non aqueux ou des solvants organiques ou des liquides lipophiles, en particulier des huiles et des huiles grasses.

17. Procédé selon l'une des revendications 1 à 16, **caractérisé en ce que** la matière solide à dissoudre est un principe actif médicamenteux, une substance active cosmétique, un additif alimentaire, un colorant ou un pigment.

18. Procédé selon l'une des revendications 1 à 17, **caractérisé en ce que** la force appliquée pour broyer les particules de la suspension est exercée par un procédé à haute pression, des homogénéisateurs du type à intervalle de piston (par exemple , APV Gaulin, NiroSoavi, Avestin), du type « à flux de Jet » (par exemple Microfluidizer) ou une presse française (SLM Instruments, Urbana, Ill. États-Unis) étant notamment utilisés.

19. Procédé selon l'une des revendications 1 à 18, **caractérisé en ce que** lors de l'utilisation d'homogénéisateurs à haute pression la pression d'homogénéisation est supérieure à 100 bar, de préférence supérieure ou égale à 500 bars, en particulier supérieure ou égale à 1500 bar et le plus préférablement supérieure ou égale à 2000 bar.

20. Procédé selon l'une des revendications 18 ou 19, **caractérisé en ce que** lors de l'utilisation d'homogénéisateurs à haute pression pour obtenir la taille moyenne des particules PCS inférieure à 1 µm, le nombre de cycles d'homogénéisation est inférieur à 10 en particulier inférieur à 5, de préférence inférieur à 3 et en particulier de seulement 1.

21. Procédé selon l'une des revendications 1 à 20, **caractérisé en ce que** la suspension de particules obtenue après le broyage des particules est utilisée directement ou après séparation des particules, en particulier dans des applications pharmaceutiques et cosmétiques, de préférence sous forme de comprimés et de capsules, de crèmes, de pommades ou de poudres pour reconstitution avant utilisation.

22. Procédé selon l'une des revendications 1 à 21, **caractérisé en ce que** la suspension obtenue est ensuite traitée pour obtenir des produits intermédiaires ou des produits finaux.

23. Procédé selon l'une des revendications 1 à 22, **caractérisé en ce que** la suspension obtenue est traitée ensuite par dépôt sur des pastilles de sucre ou par incorporation dans des pastilles de matrice.

24. Procédé selon l'une des revendications 1 à 22, **caractérisé en ce que** la suspension obtenue est lyophilisée ou séchée par pulvérisation.

25. Procédé selon l'une des revendications 1 à 22, **caractérisé en ce que** la suspension obtenue est utilisée comme liquide de granulation et un granulat qui est appliqué à des comprimés directement ou après compression est obtenue par une étape de granulation.

26. Procédé selon l'une des revendications 1 à 22, **caractérisé en ce que** des capsules de gélatine dure ou molle sont remplies avec la suspension obtenue directement ou après modification.

27. Procédé selon l'une des revendications 1 à 26, **caractérisé en ce que** l'indice de polydispersité (PI) est inférieur à 0,35, de préférence inférieur à 0,30, en particulier inférieur à 0,2, et de préférence dans la gamme de <0,2 à 0,1.
